# EUROPEAN PATENT SPECIFICATION

(11) **EP 4 616 837 B1**
(45) Date of publication and mention of the grant of the patent: **04.11.2026**
(21) Application number: 24382266.5
(22) Date of filing: 12.03.2024
(51) Int. Cl.: A61H 1/02

(54) **SYSTEM FOR CONTROLLING A ONE-LEG EXOSKELETON USING COMPUTER VISION**
SYSTEM ZUR STEUERUNG EINES EINBEINIGEN EXOSKELETTS MITTELS COMPUTERSICHT
SYSTÈME DE COMMANDE D'EXOSQUELETTE À UN SEUL PIED PAR VISION ARTIFICIELLE

(43) Date of publication of application: 17.09.2025
(73) Proprietor: Bionic Mobility Solutions, SL, 08820 El Prat de Llobregat (Barcelona) (ES)
(72) Inventor: Martínez Villar, Iván, 08820 El Prat de Llobregat (Barcelona) (ES); Belda Lois, Juan Manuel, 46022 Valencia (ES); Lamas Rodríguez, Mario, 46022 Valencia (ES)
(74) Representative: Clarke, Modet y Cía., S.L.

(56) References cited:
- US-A1- 2015 134 079
- US-A1- 2022 362 094

## Description

### FIELD OF THE INVENTION

This invention belongs to the area of orthotic devices and gait assistance. In particular, it relates to control of movements of an exoskeleton for only one leg.

### BACKGROUND OF THE INVENTION

Many health conditions (e. g. stroke or injury) produce a form of gait impairment in which an intact mobility of one of the two lower extremities is maintained. For instance, many people suffering from severe hemiplegia or hemiparesis have this form of gait impairment. Usually, they can maintain balance while standing unaided and some can even walk with great difficulty. At present, many orthotic devices for one leg intended for these people are mostly mechanical and do not include electronic means. More advanced orthotic devices like exoskeletons, sometimes called robotic orthotic devices or simply robots, include electro-mechanical means for performing and/or supervising assistance operations. However, they are mostly intended for two legs. As a result, the gait impairment demands of this population with only one affected leg are poorly addressed.

### BRIEF DESCRIPTION OF THE INVENTION

The present invention is realised by the features of claim 1 and is conceived because of the limitations of the prior art. In particular, it aims at allowing users to walk for long periods, safely and without excessive effort.

Due to the asymmetric mobility performance of the potential users, there are many considerations to take into account when operating a one-leg exoskeleton. Among others compensation for certain movements is required depending on the phase of the gait in progress, gait patterns of users may vary greatly, and the presence of terrain irregularities during different phases of the gait may require selective responses within a short time. For these tasks, inferring the movement intention of the user allows gain time and take preventive measures when terrain hazards are identified, such as obstacles or problematic surfaces for using the exoskeleton.

An object of the present invention is a system for controlling an exoskeleton of one leg. The system can cooperate with the exoskeleton that comprises articulated units movable by a first actuator and a second actuator. The system comprises several inertial mass units and pressure sensors to collect gait information. The system further comprises a computer vision unit to collect environment information associated to a user path and to detect a terrain hazard and its geometrical attributes. A user gait phase is inferred via a processor unit comprised in the system using additional gait information collected from the actuators. A response is produced with selective instructions for the actuators, based, at least, on the geometrical attributes of the terrain hazard, a user profile with user limitations and a user intention obtainable from interfaces or inferred by processing pressure sensors and IMUs information.

### BRIEF DESCRIPTION OF THE FIGURES

The implementations of the present invention are described, by way of example only, in the attached illustrations in which the similar elements are numbered in the same way in the various figures:
FIG. 1: Schematic block diagram of the system according to the present invention.
FIG. 2A: Lateral view of an example of an exoskeleton of one leg where the present invention can be used. FIG. 2B: Lateral view of an example of an exoskeleton of one leg where the present invention can be used and its quadripod stick. FIG. 2C Body planes and body axes.
FIG. 3A: Phases of an ordinary gait cycle. FIG. 3B: Graph representing hip flexion/extension during an ordinary gait cycle. FIG. 3C: Graph representing knee and hip flexion/extension during an ordinary gait cycle.
FIG. 4: Example table with a list of terrain hazards identifiable using a computer vision unit.
FIG. 5: Schematic block diagram of a locomotion strategy implementation in a system according to the present invention to control an exoskeleton.
FIG. 6: Example table with information of a user profile.
FIG. 7: Practical example of a terrain detection and response using a locomotion strategy in a system according to the present invention to control an exoskeleton.

### Numerical references glossary:

100 System.
102 User
110 Computer vision unit.
112 Al computing unit.
118 Camera.
120 Processor unit.
124 User profile.
190 Locomotion strategy .
200 Exoskeleton.
202 Hip unit.
204 Femur unit.
204a Femur extension.
206 Tibia-peroneal unit.
206a Tibia-peroneal extension.
208 Foot unit.
210 First actuator.
220 Second actuator.
230 Adjusting strap.
240 Back unit.
242 Battery.
250 Waist unit.
260 Exoskeleton-embedded interface.
270 User interface
272 Quadripod stick
302 Back IMU
304 Femur IMU.
306 Tibia-peroneal IMU.
308 Foot pressure sensors.

### DESCRIPTION OF METHODS OF IMPLEMENTATION

Referring to the above figures, without being restrictive, various implementations of the invention are presented for a better understanding.

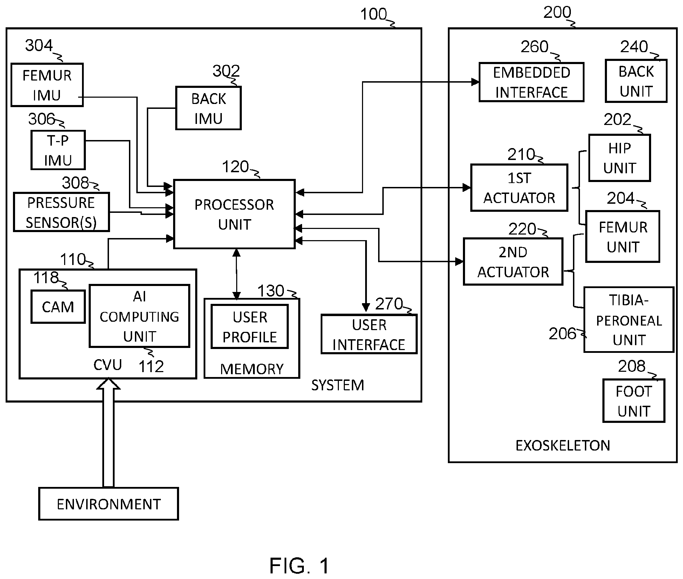

**FIG. 1** schematically shows a system 100 for controlling movements of an exoskeleton 200 to gait assistance for a user wearing it.

Some type of exoskeletons 200 have driven joints in articulated members via actuators, included in the exo-leg. In the present example, there is a first actuator 210 for moving a hip unit 202 with respect to a femur unit 204, a second actuator 220 for moving the femur unit 204 with respect to a tibia-peroneal unit 206. The proposed system 100 may couple with these existing actuators 210, 220 for gait assistance. In case of an exoskeleton 200 does not include them or cannot be coupled to the system 100, the exoskeleton should be modified to install controllable actuators 210, 220. Without limitation, the FIG. 1 assumes existing actuators 210, 220 of the exoskeleton 200 are compatible with the system 100.

To provide proper assistance to the user, the system 100 detects whether there is a hazard terrain based on environment information, and decides a response based on current user gait phase, user intention of movement and user profile 124 previously stored in a memory 130. An important aspect to achieve this aim is a computer vision unit 110 to collect environment information for the system 100 to avoid or mitigate risks and take preliminary measures by modifying the operation of the exoskeleton 200 accordingly. The computer vision unit 110 may include an artificial intelligence (AI) computing unit 112 and one or more cameras 118 equipped with depth sensing technology to allow capturing depth data usually using the infrared spectrum (for example, Intel RealSense). The camera 118 can provide terrain geometrical attributes of a path or region where the user may walk. Terrain geometrical attributes are typically inclination or irregularities of the terrain surface and the depth, width, height of objects on the terrain.

The computer vision unit 110 includes an algorithm that can be trained by machine learning and can classify the images from the environment nearby.

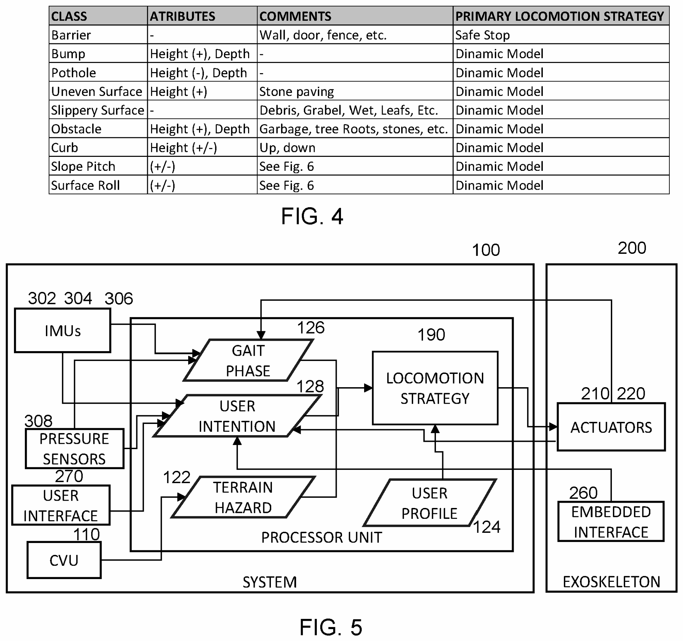

In an embodiment, there are defined nine different terrain hazards (see FIG. 4). Some terrain irregularities and little obstacles as for example, ramps, curbs, bumps, potholes, stones, branches or wet surfaces on the path to be followed by the exoskeleton 200 might represent a safety concern or a source of discomfort for the user. In that case, the system 100 may activate a selective response for the actuators 210, 220. Tests performed show the computer vision unit 110 is very reliable and can classify these nine terrain hazards in nearly real time (typically, less than 200ms) and with a high accuracy (typically, greater than 95%).

The system 100 may operate following a hierarchical scheme with different layers and its corresponding functions. For instance, the system 100 may have three layers. A high-level layer of control is to decide on a locomotion strategy with the aid of different information including that from a 3D space region peripherical to the user, typically the range of vision is 60° vertical, 90° horizontal with a depth of between 0,2cm to 4m). Each locomotion strategy involves determining the biomechanics of the movements necessary to react to terrain irregularities, barriers and obstacles considered and the corresponding kinematics. i.e. defining the movement patterns, positions, velocities, accelerations, and trajectories of the body segments and joints needed to execute such strategy.

Once the strategy is determined, the system 100 uses a mid-level and a low-level layers of control in charge of calculating the dynamic equations and the corresponding joint positions, velocities and torques that the actuators should follow and control its execution.

Terrain hazards such as stairs, walls, doors, big moving objects are also included into the training process of the algorithm, typically executed by the Al computing unit 112 (As NVIDIA Nano Jetson Orin) of the computer vision unit 110. Sometimes, particular terrain geometrical attributes as an excessive height, depth, length or inclination may imply producing a direct stop response for the exoskeleton 200 (e. g. a wall on the path).

To preserve safety and comfort, the kinematics of articulated units 202, 204, 206 are influenced by the user profile (e.g. age, anthropometry, health condition, gait pattern setup, fitting, etc.). Key parameters like toe and heel clearance or balance on an inclined surface are very influenced by age and physical condition and may have a big impact on the locomotion strategy (in general the older and the worse the condition, the lower the toe and heel clearance and the worse the balance achievable). Other parameters like walking speed (defined in the gait pattern setup of the user profile by the time of step) may also have a strong influence on the locomotion strategy as the latency to implement some responses it may be too long for safe and/or comfortable execution.

Another important aspect of system 100 is the execution of an algorithm for inferring the gait phase and an algorithm for inferring user intention of subsequent movements. Inputs for inferring the gait phase with this algorithm are sensor data from the exo-leg associated with the kinematics of the disabled side and the pelvis and data from the actuators. The details of the algorithm for inferring user intention is out of the scope of the present disclosure.

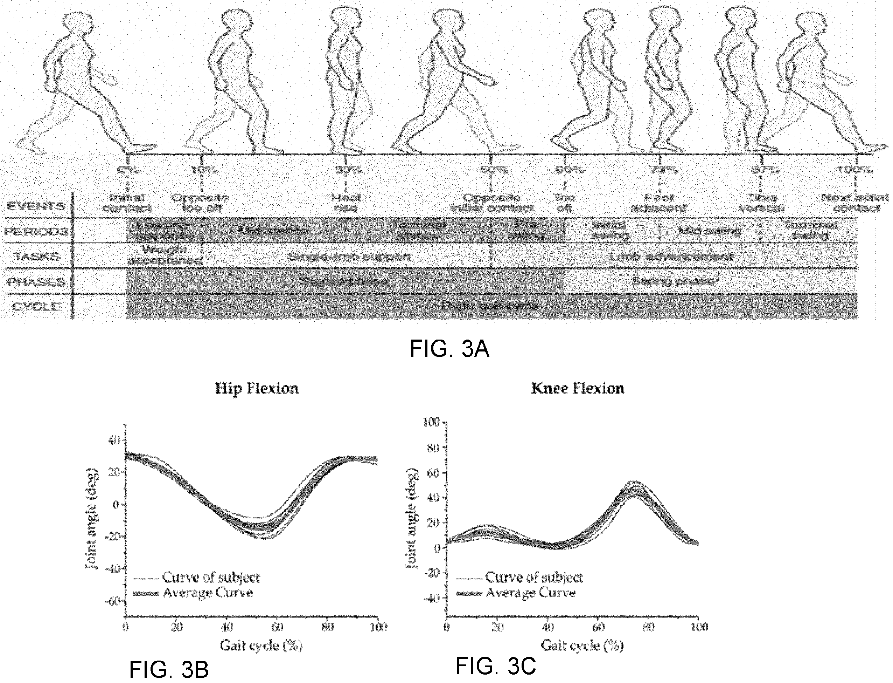

More in particular, sensor data are obtained mainly via the femur IMU 304 located on the femur unit and tibia-peroneal IMU 306 located on the tibia-peroneal unit, and back IMU 302 located in a back unit 240 and also from the pressure sensors 308 located on a foot unit 208 (on the sole). The outputs of the algorithm are classified into 7 classes corresponding to 7 ordinary gait periods: loading response, mid stance, terminal stance, pre-swing, initial swing, mid-swing, and terminal swing, as shown in FIG. 3A.

On top of that, the algorithm may provide 2 additional classes related to the user intent of movement depending if he/she wants to keep on walking or to stop: Go, Stop. The back IMU 302 located on the back unit 240 close to the pelvic area is the main contributor to it.

The system 100 also takes into account input signals received from one or more interfaces 260, 270 to follow a predefined order, which can be as example: go, stop, sit-down, or stand-up and that, - unless unsafe- has priority over other automatic responses calculated by the system 100.

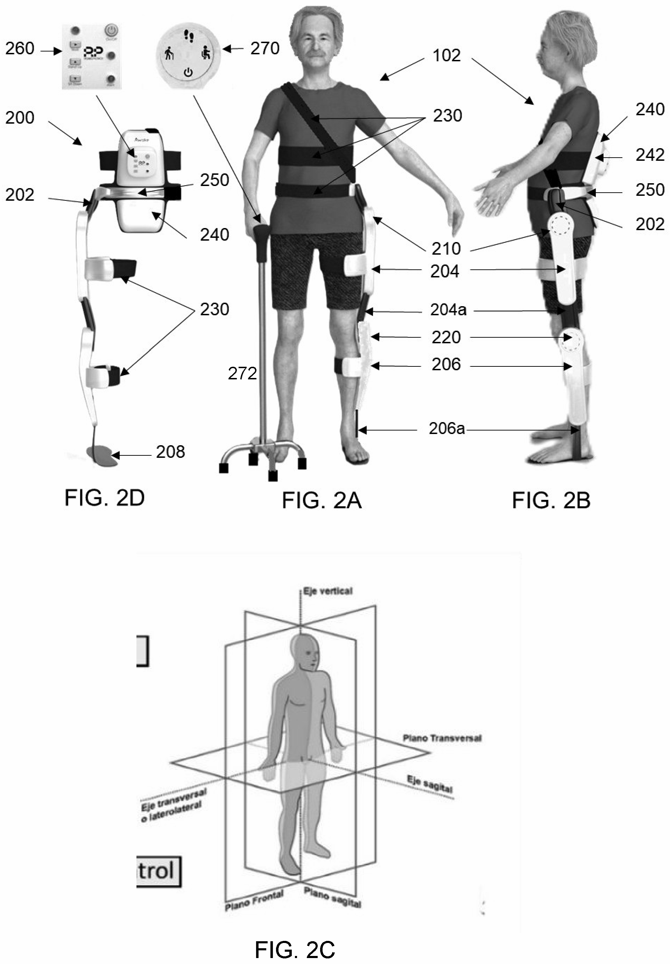

**FIG. 2A** shows a lateral view of a one-leg exoskeleton 200 wore by a user 102 that can install an embodiment of system for controlling it according to the present invention. FIG. 2B shows a lateral view of the same exoskeleton 200. Both figures will be described together along with FIG. 2D that shows the same exoskeleton alone without the user.

The exoskeleton 200 is an orthotic robotic structure for moving one user leg in a gait-like motion that emulates human gait. The exoskeleton 200 is anatomically conceived and is intended to be attached or secured to the user body, over the clothing, typically, using adjusting straps 230 at the leg, waist and, preferably at the torso and/or shoulder of the user 102, as well. The exoskeleton 200 includes several articulate units and non-articulate units, some of the articulate units are motor driven via actuators. The user is responsible of changing the direction since the exoskeleton is preferably devised to assist in moving forward (sagittal axis)

The example exoskeleton 200 of FIGs. 2A-2B and FIG. 2D includes a waist unit 250, a back unit 240, a hip unit 202, a femur unit 204, a tibia-peroneal unit 206, and a foot unit 208. The exoskeleton 200 includes a first actuator 210 for moving the femur unit 204 relative to the hip unit 202. The exoskeleton 200 includes a second actuator 220 for moving the tibia-peroneal unit 206 relative to the femur unit 204. The foot unit 208 can move relative to the tibia-peroneal unit 206 with an actuator in other embodiments. To save weight the present example exoskeleton 200 does not include a third actuator for the user ankle. However, the length between the foot unit 208 and the tibia-peroneal unit 206 can be adjusted and set according to the user 102 anatomy and needs. Optionally, the foot unit 208 can be modified to include a resistant but elastic material (e.g. carbon fiber) to act as a spring that accumulates energy during the stance phase bringing it back at the beginning of the swing. The user foot can move within the foot unit 206 according to the gait motion. Some units may be extendable to adjust different users' heights. In this embodiment, there are a femur extension 204a and a tibia-peroneal extension 206a typically realized as a telescopic mechanism and a foot extension.

The exoskeleton 200 allows rotation of the femur unit 204 and tibia-peroneal unit 206 in a sagittal plane. The sagittal plane is an anatomical plane that divides the body into right and left sections. It is perpendicular to the transverse and frontal planes as shown in Fig. 2C along with anatomical axes.

The system controls the exoskeleton to allows motion between articulate units to be electronically and/or mechanically limited within a range so that safety for the user 102 is ensured. Typically, a first range between 15 degrees extension and 120 degrees flexion for the hip, and 0° extension and 90° flexion for the knee. These ranges can be individually selected as part of the setup.

The articulate movements are electronically controlled by a system 100 according to information gathered by several sensors (e.g. IMU, pressure sensors for the sole) and by a computer vision unit, also by a gait phase and user intention module and by interfaces. A gait pattern can be predefined and customizable for each user according to his/her needs and preferences (e.g. health condition). The gait pattern can also be adapted in real-time to the user movement intention, user command, or to another locomotion strategy (e.g. in case of a terrain hazard detected).

In this embodiment, there are three inertial mass units, IMUs. A back IMU 302, a femur IMU 304 located on the femur unit 204, and a tibia-peroneal IMU 306 located on the tibial/peroneal unit 206. Several pressure sensors 308 are also attached to the sole of the foot unit 208. These are considered the basic sensors that in cooperation with a computer vision unit and with a processor unit allow to interact with the environment, thus the exoskeleton operations during a gait cycle are monitored and/or modified when needed.

In this embodiment, each actuator 210, 220 of the exoskeleton 200 includes a DC motor, gearbox, control electronics, and one or more encoders. The actuators 210, 220 are connected to both a power supply (e.g. a DC battery 242) and the processor unit via signal wiring and power wiring. The power supply is typically located on a back unit 240 of the exoskeleton 200. The processor unit typically includes a microprocessor and communication cards (Bluetooth, WiFi, etc.). An embedded interface 260 located on a back unit 240 may be used to operate the exoskeleton 200 by an assisting person.

A user interface 270 can also be provided to the user to operate the exoskeleton 200. For instance, in some embodiments, the user interface may be an app installed in a mobile phone and communicate via Bluetooth or other wireless protocol. Additionally or alternatively, in some embodiments, the exoskeleton 200 may be associated with a quadripod stick 272 for the user to hold when standing up or walking. The quadripod stick may include the user interface in the form of a keypad and a display in the handle and communicate via Bluetooth or other wireless protocol.

The user interface 270 and embedded interface 260 allow to operation of the basic motion or general functions such as stopping, walking, sitting down, standing up, also system status like battery level, or failure, etc. The user interface may include an on/off switch, emergency stop buttons, LEDs, buzzers, and haptic indicators. It is important to have bidirectional communication between the exoskeleton 200 and the user so that both work collaboratively. The exoskeleton 200 may communicate an intended response to the user to avoid surprises and improve cooperation. An example is when the computer vision unit 110 detects an obstacle, and the exoskeleton 200 goes into obstacle avoidance response while the obstacle has gone unnoticed by the user. Therefore, it is important to provide information in a non-intrusive way so that the system 100 be updated to abort the unnecessary obstacle avoidance response. Ideally, communication can use various channels to ensure the reception of information and make it as non-intrusive as possible. Preferably, combined haptic-audio and/or haptic-visual strategies can be employed.

Another example scenario is the user-facing a curb. As soon as the camera identifies the curb, a vibration occurs (so the user be aware that there is a possible change in kinematics) and then the user may decide to look to confirm the detection of the curb. According to the user preferences, the system 100 can be configured to require or not a user confirmation prior to execute some responses in the exoskeleton.

**FIG. 3A** shows the phases of an ordinary gait cycle according to a conventional gait model. The gait cycle is measured from one-foot strike to the subsequent foot strike of the same foot. The gait cycle can be broken down into the stance phase and swing phase, which alternate for each leg. During a stance left phase a left foot is on the ground the entire time. During a swing right phase, the right foot is in the air the entire time. Likewise, for the opposite foot, there is a stance right phase and a swing left phase. Thus, during a stance right phase the right foot is on the ground the entire time and during a swing left phase the left foot is in the air the entire time.

In turn, the stance phase includes several sub-phases: double support sub-phase, single support right sub-phase, double support sub-phase, and single support left sub-phase. The gait cycle has several periods: loading response, mid-stance, terminal stance, pre-swing, initial swing, mid-swing and terminal swing).

**FIG. 3B** shows a graph representing hip flexion/extension during an ordinary gait cycle. FIG. 3C shows a graph representing knee flexion/extension during an ordinary gait cycle. The processor unit of the system is configured to implement a locomotion strategy that mimics these patterns, making proper adjustments according to the user profile (e. g. anthropometry, health limitations, etc.) in normal circumstances (e. g., walking on a safe terrain) during a gait cycle.

When a terrain hazard is identified or when the user produces an instruction for the exoskeleton via an interface, the system responds to it depending on several factors. In particular, according to the particular attributes of the terrain hazard, the current gait phase, and/or the user intention, the system response may vary. The processor unit manages this response according to a locomotion strategy.

**FIG. 4** shows a table with an example list of terrain hazards in the first column that can be identified by the system according to environment information collected by the computer vision unit. The second column of the table defines attributes of the first column, and the third column offers comments with several objects that may belong to a class. The attributes indicate if there are additional variables to be considered. E.g., for a bump or a pothole it is important to consider not only the height (positive (+) for a bump and negative (-) for a pothole) but also the depth (in the walking direction) to evaluate the best strategy for the user to face it as it could be dangerous if the depth of the obstacle is longer than the length of the step the user could perform. Typically, the computer vision unit is calibrated to detect when obstacles fulfil certain criteria.

In the case of a surface, there could be a positive or negative inclination in the direction of walking (pitch: slope up/down) or lateral to it (yaw) and it could also be positive or negative (clockwise/anticlockwise).

**FIG. 5** shows a block diagram of an embodiment of the system 100 to control an exoskeleton 200. The processor unit 120 according to each terrain hazard 122 and other inputs, such as the user profile 124, the gait phase 126, and the user intention 128, produces a set of instructions for the actuators 210, 220 of the exoskeleton 200.

A common source of safety and comfort problems is the terrain, so it is discussed in more detail. The computer vision unit 110 collects environment information near a user path and it is programmed to identify whether such information matches a terrain hazard 122, such as a barrier, a bump, a pothole, an uneven surface, a slippery surface, an obstacle, a curb, a slope pitch, a surface roll. These terrain hazards can be included in a list as shown in FIG. 4, which can be updated.

When a terrain hazard is detected, a locomotion strategy algorithm 190 in the processor unit 120 is in charge of analyzing it taking into account the input of the gait phase the user intention of movement, and that of the user profile. As a result, the ordinary gait cycle movements are represented in FIGs. 3B-3C may be altered. For instance, the actuators are instructed to modify these gait patterns and follow others to prevent the user from tumbling or falling.

Each locomotion strategy involves determining the biomechanics of the movements necessary to react to terrain irregularities, barriers, and obstacles considered and the corresponding kinematics.

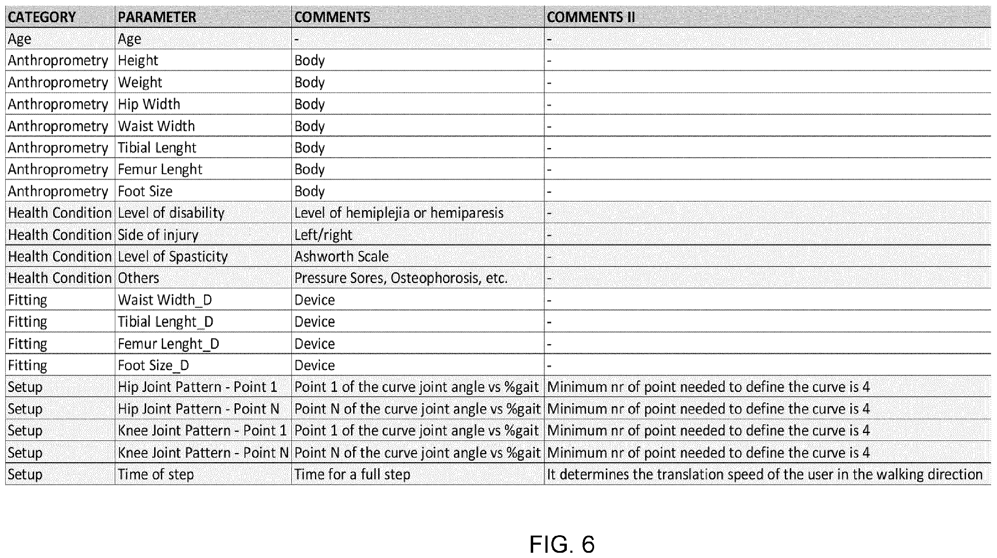

**FIG. 6** shows a table with an example of information in a user profile. Several parameters influence the suitable responses for the user that can be offered by the system in case of a particular terrain hazard. A particular key parameter is the time of step that may limit the possible responses offered by the system to the user to overcome a detected terrain hazard. This time of step is typically in the order of 700 to 900ms.

Other particular implementations and different realizations of the system are possible like the ones described above.

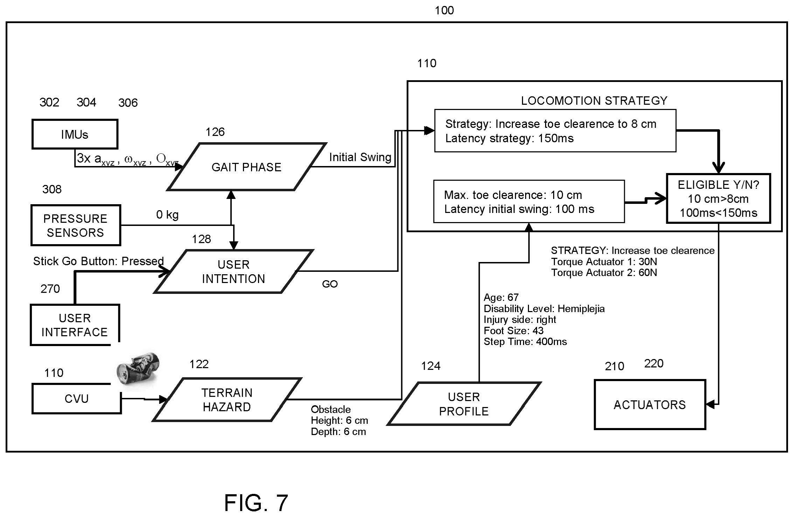

**FIG. 7** shows a practical example of operation of the system 100 when a particular obstacle is detected and the corresponding response provided.

The system 100 is coupled with a one-leg exoskeleton that a user is wearing. The one-leg exoskeleton may have similar units to the one of FIGs. 2A-2B, 2D. The system 100 (or one-leg exoskeleton itself) includes a first actuator 210 in charge of moving a hip unit with respect to a femur unit, and with a second actuator 220 in charge of moving the femur unit with respect to a tibia-peroneal unit. A processor unit 120 controls said actuators 210, 220.

The system 100 includes a computer vision unit 110 that collects environment information nearby the user. In this case, a terrain hazard 122 detected within a configurable safe range (e.g. 0,6 meter distance from the user). Let's say it is an obstacle with geometrical attributes of 6 cm height, 6 cm depth. This information is used by the processor unit 120 to produce a selective response also based on other considerations.

The user intention 128 is taken into account by the system 100 via a user interface 270, for instance, present in a handle stick (e.g. a "go" button is pushed).

The system 100 includes three IMUs 302, 304, 306 associated with the exoskeleton units and with a pressure sensor (308). They collect gait information generated by the user/exoskeleton motion, typically acceleration a_{xyz}, angular velocity w_{xyz} and position O_{xyz} in three axes for each IMU. The processor unit 120 processes gait information from the actuators 210, 220, and the IMUs 302, 304, 306 and the pressure sensor 308 and infers the gait phase 126 of the gait cycle the user is currently executing (e.g. he is in an initial swing of the gait cycle).

In order to produce a selective response valid for this particular user, the processor unit 120 processes the user profile 124 to obtain anatomical limitation information and/or physical information recorded during a setup of the system 100. This information may be general like age, disability level, age, anthropometry, health condition, injury side, foot size, or may be more specific like gait pattern setup step time (e.g., a statistical value taken from tests for the user). Note, the user age is usually employed by the system 100 to adjust the eligibility of potential responses by setting a safety margin (e.g. when a response implies to be close to a user profile value, it may fail more often in older people).

The processor unit 120 infers with the user profile a maximum toe clearance (e.g. 10 cm) and a latency for the initial swing (e.g. 100 ms).

The inputs of terrain hazard geometrical attributes, user intention, gait phase, user profile are processed according to a locomotion strategy, which can be in the form of a database in an embodiment. That is, the inputs define an output that the processor 120 transforms into a response with instructions for the actuators 210, 220.

In this case, the locomotion strategy has a latency (e.g. 150ms) and implies to increase toe clearence (e.g. up to 8 cm). The processor unit 120 determines that the terrain hazard 122 can be overcome. Consequently, it enables the locomotion strategy. As a result, a response is produced with instructions for the actuators 210, 220 (e.g., torque first actuator 30N, torque second actuator 60N).

## Claims

1. System (100) for controlling a one-leg exoskeleton (200) for walking assistance of a user (102), wherein the exoskeleton (200) comprises:
a waist unit (250), a hip unit (202), a femur unit (204), a tibia-peroneal unit (206), and a foot unit (208), wherein the hip unit (202) is movable with respect to a back unit (240), wherein the femur unit (204) is movable with respect to the hip unit (202), wherein the tibia-peroneal unit (206) is movable with respect to the femur unit (204), wherein the foot unit (208) is movable with respect to the tibia-peroneal unit (206);
wherein the exoskeleton (200) or the system (100) comprises:
a first actuator (210) for moving the femur unit (204) with respect to the hip unit (202) and a second actuator (220) for moving the tibia-peroneal unit (206) with respect to the femur unit (204);
wherein the system (100) comprises:
a plurality of inertial mass units (302,304,306) and pressure sensors (308) configured to collect a first set of gait information;
a processor unit (120) configured to collect a second set of gait information from the first actuator (210) and second actuator (220), to infer a user gait phase (126) with the first and second sets of gait information;
**characterized in that** the system (100) further comprises:
a computer vision unit (110) configured to collect environment information associated to a user path and to detect a terrain hazard (122) within a safe range, and to provide a plurality of geometrical attributes of the terrain hazard (122);
wherein the processor unit (120) is configured to process the geometrical attributes of the terrain hazard (122) together with:
a user profile (124) comprising user anatomical limitations and temporal information for gait phases of the user gait cycle and a maximum range applicable by the actuators (210, 220) when moving associated units;
wherein the processor unit (120) is configured to determine a locomotion strategy (190) comprising selective instructions for the first actuator (210) and the second actuator (220) to overcome the terrain hazard.

2. System for controlling a one-leg exoskeleton (200) according to claim 1, wherein geometrical attributes of the terrain hazard (122) obtained by the computer vision unit (110) comprise a combination of one or more: positive or negative height, depth, positive or negative inclination in sagittal plane, slope up or down, lateral clockwise or anticlockwise.

3. System for controlling a one-leg exoskeleton (200) according to any of claims 1 or 2, further comprising an interface (270, 260) for inputting a user intention (128) including stop walking and walking.

4. System for controlling a one-leg exoskeleton (200) according to any of claims 1 to 3, wherein the user intention is obtained from an interface (270, 260).

5. System for controlling a one-leg exoskeleton (200) according to any of claims 1 to 4, wherein the user intention is obtained from processing information from IMUs (302,304,306), pressure sensors (308) and actuators (210,220).

6. System for controlling a one-leg exoskeleton (200) according to any of claims 1 to 5, wherein the user profile (124) comprises at least a step time of the user gait cycle and a maximum toe clearance, so when a terrain hazard (122) is detected, the processor unit (120) is configured to determine whether a locomotion strategy (190) to overcome the terrain hazard (122) is eligible for the user (102), and to generate the selective instructions for the first actuator (210) and the second actuator (220) adjusting a toe clearance within the maximum toe clearance.

7. One-leg exoskeleton (200) comprising a system according to any of claims 1 to 6, wherein the computer vision unit (110) is previously trained by a machine learning algorithm to classify a detected terrain hazard into a barrier, a bump, a pothole, an uneven surface, a slippery surface, an obstacle, a curb, a slope pitch or a surface roll.

8. One-leg exoskeleton (200) comprising:
a waist unit (250), a hip unit (202), a femur unit (204), a tibia-peroneal unit (206), and a foot unit (208), wherein the hip unit (202) is movable with respect to the back unit (240), wherein the femur unit (204) is movable with respect to the hip unit (202), wherein the tibia-peroneal unit (206) is movable with respect to the femur unit (204), wherein the foot unit (208) is movable with respect to the tibia-peroneal unit (206);
a first actuator (210) for moving the femur unit (204) with respect to the hip unit (202) in the sagittal plane, and a second actuator (220) for moving the tibia-peroneal unit (206) with respect to the femur unit (204) in the sagittal plane;
a system according to any of claims 1 to 4, wherein the foot unit (208) comprises an elastic portion for receiving the user foot and accumulate and liberate energy during a gait cycle.

## Patentansprüche

1. System (100) zum Steuern eines einbeinigen Exoskeletts (200) zur Gehunterstützung eines Benutzers (102),
wobei das Exoskelett (200) Folgendes umfasst:
eine Tailleneinheit (250), eine Hüfteinheit (202), eine Femureinheit (204), eine Tibia-Fibula-Einheit (206) und eine Fußeinheit (208), wobei die Hüfteinheit (202) in Bezug auf eine Rückeneinheit (240) beweglich ist, wobei die Femureinheit (204) in Bezug auf die Hüfteinheit (202) beweglich ist, wobei die Tibia-Fibula-Einheit (206) in Bezug auf die Femureinheit (204) beweglich ist, wobei die Fußeinheit (208) in Bezug auf die Tibia-Fibula-Einheit (206) beweglich ist;
wobei das Exoskelett (200) oder das System (100) Folgendes umfasst:
einen ersten Aktuator (210) zum Bewegen der Femureinheit (204) in Bezug auf die Hüfteinheit (202) und einen zweiten Aktuator (220) zum Bewegen der Tibia-Fibula-Einheit (206) in Bezug auf die Femureinheit (204);
wobei das System (100) Folgendes umfasst:
eine Vielzahl von Trägheitsmasseeinheiten (302, 304, 306) und Drucksensoren (308), die dazu konfiguriert sind, einen ersten Satz von Ganginformationen zu erfassen;
eine Prozessoreinheit (120), die dazu konfiguriert ist, einen zweiten Satz von Ganginformationen von dem ersten Aktuator (210) und dem zweiten Aktuator (220) zu erfassen, um anhand des ersten und des zweiten Satzes von Ganginformationen eine Gangphase (126) des Benutzers abzuleiten;
**dadurch gekennzeichnet, dass** das System (100) ferner Folgendes umfasst:
eine Computersichteinheit (110), die dazu konfiguriert ist, Umgebungsinformationen im Zusammenhang mit einem Benutzerpfad zu erfassen, ein Geländehindernis (122) innerhalb eines sicheren Bereichs zu erkennen und eine Vielzahl von geometrischen Attributen des Geländehindernisses (122) bereitzustellen;
wobei die Prozessoreinheit (120) dazu konfiguriert ist, die geometrischen Attribute des Geländehindernisses (122) zusammen mit Folgendem zu verarbeiten:
einem Benutzerprofil (124), das anatomische Einschränkungen des Benutzers und zeitliche Informationen zu den Gangphasen des Gangzyklus des Benutzers und einen maximalen Bereich umfasst, der von den Aktuatoren (210, 220) beim Bewegen zugeordneter Einheiten angewendet werden kann;
wobei die Prozessoreinheit (120) dazu konfiguriert ist, eine Fortbewegungsstrategie (190) zu bestimmen, die selektive Anweisungen für den ersten Aktuator (210) und den zweiten Aktuator (220) umfasst, um das Geländehindernis zu überwinden.

2. System zum Steuern eines einbeinigen Exoskeletts (200) nach Anspruch 1, wobei die geometrischen Attribute des Geländehindernisses (122), die von der Computersichteinheit (110) erhalten werden, eine Kombination aus einem oder mehreren von Folgendem umfassen: positive oder negative Höhe, Tiefe, positive oder negative Neigung in der Sagittalebene, Steigung nach oben oder unten, seitliche Bewegung im Uhrzeigersinn oder gegen den Uhrzeigersinn.

3. System zum Steuern eines einbeinigen Exoskeletts (200) nach einem der Ansprüche 1 oder 2, ferner umfassend eine Schnittstelle (270, 260) zum Eingeben einer Benutzerabsicht (128), die Stoppen des Gehens und Gehen enthält.

4. System zum Steuern eines einbeinigen Exoskeletts (200) nach einem der Ansprüche 1 bis 3, wobei die Benutzerabsicht über eine Schnittstelle (270, 260) erhalten wird.

5. System zum Steuern eines einbeinigen Exoskeletts (200) nach einem der Ansprüche 1 bis 4, wobei die Benutzerabsicht durch das Verarbeiten von Informationen aus IMUs (302, 304, 306), Drucksensoren (308) und Aktuatoren (210, 220) erhalten wird.

6. System zum Steuern eines einbeinigen Exoskeletts (200) nach einem der Ansprüche 1 bis 5, wobei das Benutzerprofil (124) mindestens eine Schrittzeit des Gangzyklus des Benutzers und einen maximalen Zehenabstand umfasst, sodass, wenn ein Geländehindernis (122) erkannt wird, die Prozessoreinheit (120) dazu konfiguriert ist, zu bestimmen, ob eine Fortbewegungsstrategie (190) zur Überwindung des Geländehindernisses (122) für den Benutzer (102) in Frage kommt, und die selektiven Anweisungen für den ersten Aktuator (210) und den zweiten Aktuator (220) zu generieren, wodurch der Zehenabstand innerhalb des maximalen Zehenabstands angepasst wird.

7. Einbeiniges Exoskelett (200), umfassend ein System nach einem der Ansprüche 1 bis 6, wobei die Computersichteinheit (110) vorher durch einen Maschinenlernalgorithmus trainiert wurde, um ein erkanntes Geländehindernis als eine Barriere, eine Erhebung, ein Schlagloch, eine unebene Oberfläche, eine rutschige Oberfläche, ein Hindernis, einen Bordstein, eine Neigung oder eine Oberflächenrolle zu klassifizieren.

8. Einbeiniges Exoskelett (200), umfassend:
eine Tailleneinheit (250), eine Hüfteinheit (202), eine Femureinheit (204), eine Tibia-Fibula-Einheit (206) und eine Fußeinheit (208), wobei die Hüfteinheit (202) in Bezug auf die Rückeneinheit (240) beweglich ist, wobei die Femureinheit (204) in Bezug auf die Hüfteinheit (202) beweglich ist, wobei die Tibia-Fibula-Einheit (206) in Bezug auf die Femureinheit (204) beweglich ist, wobei die Fußeinheit (208) in Bezug auf die Tibia-Fibula-Einheit (206) beweglich ist;
einen ersten Aktuator (210) zum Bewegen der Femureinheit (204) in Bezug auf die Hüfteinheit (202) in der Sagittalebene und einen zweiten Aktuator (220) zum Bewegen der Tibia-Fibula-Einheit (206) in Bezug auf die Femureinheit (204) in der Sagittalebene;
ein System nach einem der Ansprüche 1 bis 4, wobei die Fußeinheit (208) einen elastischen Abschnitt zum Aufnehmen des Fußes des Benutzers umfasst und Energie während eines Gangzyklus speichert und freisetzt.

## Revendications

1. Système (100) de commande d'un exosquelette pour une seule jambe (200) destiné à assister la marche d'un utilisateur (102),
dans lequel l'exosquelette (200) comprend :
une unité de taille (250), une unité de hanche (202), une unité de fémur (204), une unité tibio-péronière (206) et une unité de pied (208), dans lequel l'unité de hanche (202) est mobile par rapport à une unité dorsale (240), dans lequel l'unité de fémur (204) est mobile par rapport à l'unité de hanche (202), dans lequel l'unité tibio-péronière (206) est mobile par rapport à l'unité de fémur (204), dans lequel l'unité de pied (208) est mobile par rapport à l'unité tibio-péronière (206) ;
dans lequel l'exosquelette (200) ou le système (100) comprend :
un premier actionneur (210) destiné à déplacer l'unité de fémur (204) par rapport à l'unité de hanche (202) et un deuxième actionneur (220) destiné à déplacer l'unité tibio-péronière (206) par rapport à l'unité de fémur (204) ;
dans lequel l' système (100) comprend :
une pluralité d'unités de masse inertielle (302,304,306) et de capteurs de pression (308) configurés pour recueillir un premier ensemble d'informations de marche ;
une unité de traitement (120) configurée pour recueillir un deuxième ensemble d'informations de marche auprès du premier actionneur (210) et du deuxième actionneur (220), afin de déduire une phase de marche de l'utilisateur (126) à partir des premier et deuxième ensembles d'informations de marche ;
**caractérisé en ce que** le système (100) comprend en outre :
une unité de vision par ordinateur (110) configurée pour recueillir des informations sur l'environnement associées à un trajet de l'utilisateur et pour détecter un danger lié au terrain (122) dans une portée de sécurité, et pour fournir une pluralité d'attributs géométriques du danger lié au terrain (122) ;
dans lequel l'unité de traitement (120) est configurée pour traiter les attributs géométriques du danger lié au terrain (122) conjointement avec :
un profil d'utilisateur (124) comprenant des limitations anatomiques de l'utilisateur et des informations temporelles relatives aux phases de marche du cycle de marche de l'utilisateur, ainsi qu'une amplitude maximale applicable par les actionneurs (210, 220) lors du déplacement des unités associées ;
dans lequel l'unité de traitement (120) est configurée pour déterminer une stratégie de locomotion (190) comprenant des instructions sélectives destinées au premier actionneur (210) et au deuxième actionneur (220) pour franchir le danger lié au terrain.

2. Système de commande d'un exosquelette pour une seule jambe (200) selon la revendication 1, dans lequel les attributs géométriques du danger lié au terrain (122) obtenus par l'unité de vision par ordinateur (110) comprennent une combinaison d'un ou plusieurs des éléments suivants : hauteur positive ou négative, profondeur, inclinaison positive ou négative dans le plan sagittal, pente ascendante ou descendante, orientation latérale dans le sens horaire ou antihoraire.

3. Système de commande d'un exosquelette pour une seule jambe (200) selon l'une quelconque des revendications 1 ou 2, comprenant en outre une interface (270, 260) destinée à saisir une intention de l'utilisateur (128) comprenant l'arrêt de la marche et la marche.

4. Système de commande d'un exosquelette pour une seule jambe (200) selon l'une quelconque des revendications 1 à 3, dans lequel l'intention de l'utilisateur est obtenue à partir d'une interface (270, 260).

5. Système de commande d'un exosquelette pour une seule jambe (200) selon l'une quelconque des revendications 1 à 4, dans lequel l'intention de l'utilisateur est obtenue par traitement d'informations provenant des IMUs (302,304,306), des capteurs de pression (308) et des actionneurs (210,220).

6. Système de commande d'un exosquelette pour une seule jambe (200) selon l'une quelconque des revendications 1 à 5, dans lequel le profil d'utilisateur (124) comprend au moins une durée de pas du cycle de marche de l'utilisateur et une hauteur maximale de dégagement de la pointe du pied, de sorte que, lorsqu'un danger lié au terrain (122) est détecté, l'unité de traitement (120) est configurée pour déterminer si une stratégie de locomotion (190) permettant de franchir le danger lié au terrain (122) est admissible pour l'utilisateur (102), et pour générer les instructions sélectives destinées au premier actionneur (210) et au deuxième actionneur (220) en ajustant une hauteur de dégagement de la pointe du pied dans la limite de la hauteur maximale de dégagement de la pointe du pied.

7. Exosquelette pour une seule jambe (200) comprenant un système selon l'une quelconque des revendications 1 à 6, dans lequel l'unité de vision par ordinateur (110) est préalablement entraînée au moyen d'un algorithme d'apprentissage automatique pour classer un danger lié au terrain détecté comme une barrière, une bosse, un nid-de-poule, une surface irrégulière, une surface glissante, un obstacle, une bordure, une inclinaison longitudinale de pente ou une inclinaison transversale de surface.

8. Exosquelette pour une seule jambe (200) comprenant :
une unité de taille (250), une unité de hanche (202), une unité de fémur (204), une unité tibio-péronière (206) et une unité de pied (208), dans lequel l'unité de hanche (202) est mobile par rapport à l'unité dorsale (240), dans lequel l'unité de fémur (204) est mobile par rapport à l'unité de hanche (202), dans lequel l'unité tibio-péronière (206) est mobile par rapport à l'unité de fémur (204), dans lequel l'unité de pied (208) est mobile par rapport à l'unité tibio-péronière (206) ;
un premier actionneur (210) destiné à déplacer l'unité de fémur (204) par rapport à l'unité de hanche (202) dans le plan sagittal, et un deuxième actionneur (220) destiné à déplacer l'unité tibio-péronière (206) par rapport à l'unité de fémur (204) dans le plan sagittal ;
un système selon l'une quelconque des revendications 1 à 4, dans lequel l'unité de pied (208) comprend une partie élastique destinée à recevoir le pied de l'utilisateur et à accumuler et libérer de l'énergie pendant un cycle de marche.
